# EUROPEAN PATENT APPLICATION

(11) **EP 1 754 505 A1**
(43) Date of publication of application: **21.02.2007**
(21) Application number: 05107629.7
(22) Date of filing: 19.08.2005
(51) Int. Cl.: A61M 39/22, A61M 5/145

(54) **Syringe pump**

(71) Applicant: The Automation Partnership (Cambridge) Limited, Royston, Hertfordshire SG8 5WY (GB)
(72) Inventor: Bargh, Adrian Neil, SG8 5WY, Royston, Hertfordshire (GB)
(74) Representative: Brunner, Michael John

(57) **Abstract**

A syringe pump 1 comprises a barrel 4 and a movable plunger 12, a valve 15 provided at one end of the barrel, a first port 33 between the valve and the barrel, a second port 34 between the valve and the barrel, and a valve member 20. The valve has a first inlet/outlet 17, a second inlet/outlet 18. The valve member 20 includes at least one valve passage 16 configured to connect the first and second ports selectively to the first and second inlet/outlets depending on the position of the valve member.

## Description

The present invention relates to an improved syringe pump that enables faster, more reliable priming.

A conventional syringe pump for dispensing small volumes of liquid is mounted vertically with a three port rotary valve at the top of a syringe chamber and a plunger at the bottom. In use, the plunger is drawn downwards to draw liquid into the syringe chamber from a reservoir. The aim of mounting a syringe vertically is that it should help to remove air from the syringe as air bubbles rise naturally towards the valve during priming. The valve can be switched to connect the syringe chamber to either an input port or an output port. An inherent problem with the configuration of a valve with a single port between the barrel and the valve is that there is a dead volume between the syringe barrel and the valve itself. This dead volume results in trapped air being unable to exit the syringe. When the plunger is at the top of its stroke, the air is pushed into the dead volume. However, when the syringe is refilled, because the refilling occurs through the same inlet, the trapped air is pushed back into the syringe ahead of the inflowing fluid. Thus, the configuration of such syringe pumps results in air bubbles becoming trapped and only being persuaded to leave the syringe barrel by repeated aspirate and dispense cycles and manually tapping the syringe as well. Because air is compressible, air bubbles in the syringe can cause the performance to be reduced to a level at which accuracy and reliability of pumping of the liquid is not acceptable, as a result of the fact that the air is much more easily compressed than the liquid to be pumped.

Another problem that is common is such syringes is the formation of a small slug of air in the barrel. This is particularly noticeable in syringes with small bores where the surface tension forces are sufficient to allow bubbles to remain trapped against the curved wall of the barrel.

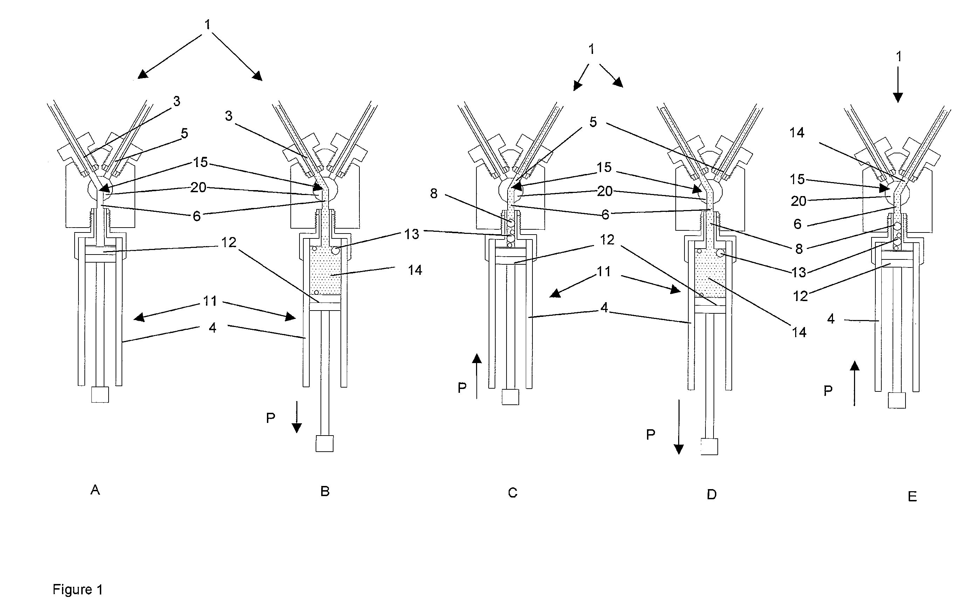

A typical syringe pump 1 and valve 2 are shown in Figures 1A - 1 E. The valve 15 consists of two ports 3, 5 through which liquid can be introduced into and removed from the syringe pump 1 and a single port 6 between a syringe barrel 4 and a valve member 20. These figures show the syringe pump 1 being filled and emptied. The syringe pump 1 has a syringe 11 having a plunger 12 with conventional seals and capable of movement within a barrel 4. Figure 1A shows the pump 1 when it is not in use and the syringe 11 is empty of liquid. Figure 1B shows the syringe 11 being filled, the plunger 12 moving in the direction of arrow P, the liquid 14 travelling through the inlet port 3, the valve member 20 and the single port 6 into the syringe barrel 4. A number of air bubbles 13 are shown in the syringe 11. Figure 1C shows the syringe 11 dispensing through the outlet port 5 via the valve 15. It shows that once the plunger 12 is at the top of its stroke and the syringe 11 is empty, the air bubbles 13 have moved out into a dead volume 8 that lies between the top of the syringe 11 and the valve 15 and are effectively trapped there. Because this dead volume 8 is part of the fluid passageway from the inlet port 3 into the syringe 11 then, as shown in Figure 1D, when the syringe 11 is refilled through the inlet port, the air bubbles 13 are forced back into the syringe 11 ahead of the incoming liquid 14. When the syringe 11 is emptied, as shown in Figure 1E, the air bubbles 13 are, again, transferred from the barrel 4 to the dead volume 8.

According to the present invention there is provided a syringe pump comprising: a barrel and a movable plunger, a valve provided at one end of the barrel, a first port between the valve and the barrel, a second port between the valve and the barrel, and wherein the valve has a first inlet/outlet, a second inlet/outlet; and a valve member including at least one valve passage configured to connect the first and second ports selectively to the first and second inlet/outlets depending on the position of the valve member.

The valve member may be a rotary valve member and the valve passage may be located across or offset from the rotational axis of the valve member. The valve member may further comprise a second passage offset from the rotational axis of the valve member. The passages may be parallel or non-parallel to one another. The passage or passages may be bores or recesses formed as cut-away portions in the side of the valve member. The valve member may be axially movable.

The syringe pump may be incorporated in a dispensing assembly that further comprises a dispenser having: a dispensing tip connected to the outlet of the or each syringe pump.

A plurality of syringe pumps may be incorporated into a dispensing array wherein the dispensing tips and syringe plungers are preferably configured to form substantially parallel arrays. The dispensing array may further comprise an actuator configured to actuate the syringe plungers simultaneously.

Examples of dispensers according to the present invention will now be further described with reference to the accompanying drawings in which:
Figures 2 to 6 show different examples of syringes according to the invention;
Figures 7a - e show the sequence of operation of the example of the invention shown in Figure 5;
Figures 8 and 9 show two different methods of attaching the syringe shown in Figure 5 to a valve block;
Figure 10 and 11 show dispensers including the syringe shown in Figure 5;
Figure 12 shows a dispensing array including a plurality of syringe pumps according to the invention.

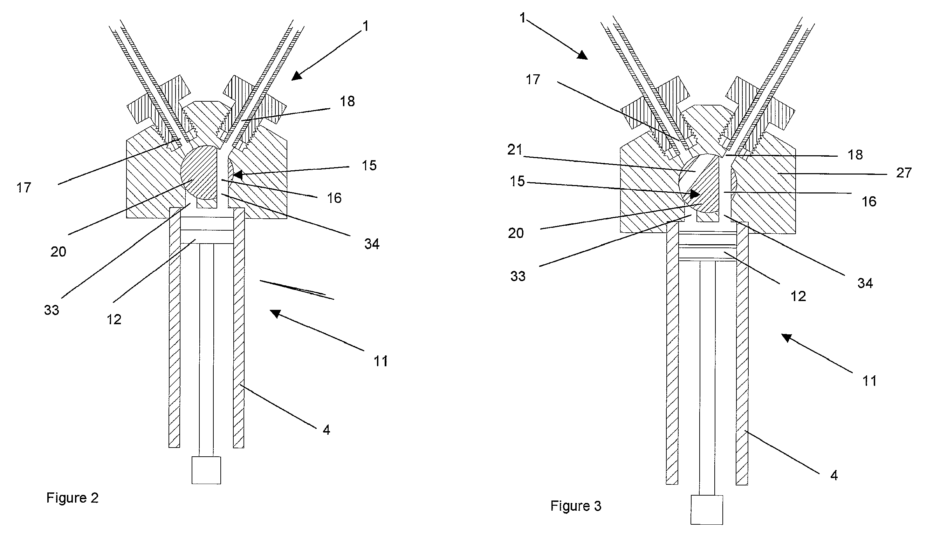

Figures 2 to 6 show different examples of syringe pumps 1 according to the present invention. Each syringe pump 1 has a syringe 11 which consists of a glass barrel 4 and a syringe plunger 12. Each syringe 11 is closed by a valve 15 that is mounted in a valve block 27. Two ports 33, 34 extend from the glass barrel 4, through the valve block 27 to connect the volume within the syringe pump 1 to a valve member 20. Two inlet/outlets 17, 18 are provided to introduce liquid into, and remove liquid from, the syringe 11. Liquid may be introduced through either inlet/outlet 17 or 18. Equally, liquid can exit through either inlet/outlet 17 or 18 depending on the valve position.

Figure 2 shows a first syringe pump 1 with a valve 15 which has a two-port rotary valve member 20 through which a single passage in the form of a bore 16 extends. The bore 16 is offset from the rotational axis of the valve member 20. In use, it allows the flow of fluid into or out of the syringe 11. The off-axis positioning of the bore 16 allows the same bore to access, sequentially on rotation, inlet/outlet 17 and port 33 and then inlet/outlet 18 and port 34.

A second syringe pump 1 shown in Figure 3 comprises, in addition to those features discussed above with regard to Figure 2, a second off-axis bore 21. The second bore 21 is at an acute angle to the first bore 16 in this example. This configuration of bores 16 and 21 enables the syringe 11 to be filled and emptied through separate bores thus ensuring that no air bubbles can remain in the syringe.

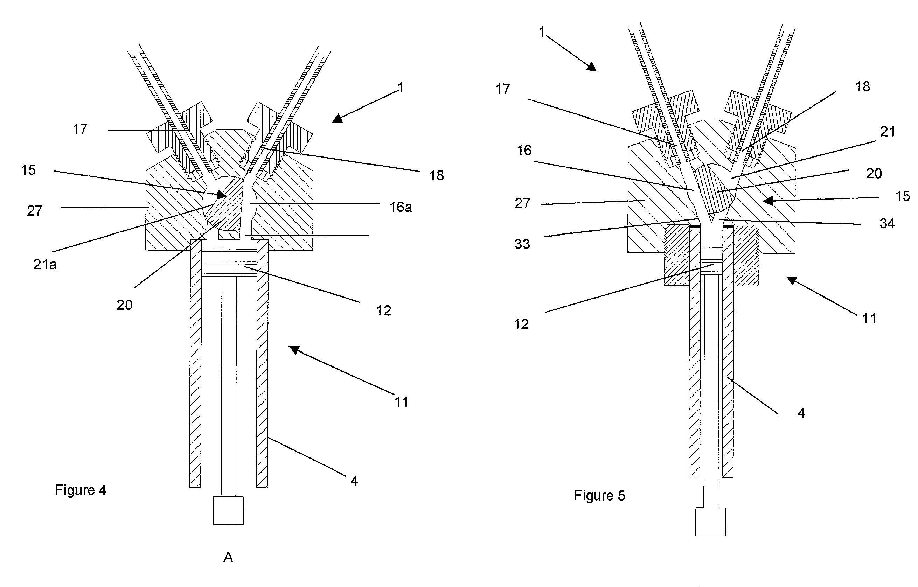

The syringe pump 1 shown in Figure 4 has a similar valve configuration to that shown in Figure 3. However, instead of bores, the sides of the valve member 20 are cut away in order to form the fluid passages. These cutaway passages 16a, 21a are easier to manufacture than the bores 16, 21 described above.

Figure 5 shows a valve 15 and associated ports 33, 34 configured for a syringe 11 with a narrow barrel 4 that would be too small to accommodate the arrangement of ports shown in Figure 4. In this example the ports 33, 34 are non-parallel and join together at the point of entry of the ports into the barrel 4.

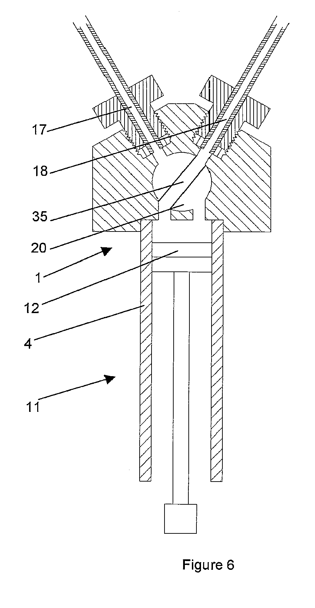

Figure 6 shows a further syringe 11 in which the valve 15 has a single, bore 35 located on across the rotational axis of the valve member 20. This syringe 11 works in a similar manner to that shown in Figure 2 in that the bore 35 connects sequentially with the ports 33, 34 and the inlet/outlets 17, 18. However, as the bore 35 is axial it links the port 33 to the opposing inlet/outlet 18 or the port 34 to the inlet/outlet 17.

The valves 15 shown in Figures 2 to 6 are rotary valves. However, the skilled man would readily understand that the principles of the present invention could be implemented with a slide or shuttle valve or any other type of valve capable of interfacing with the other integers of the syringe pump 1 described above.

Each of the syringe pumps 1 shown in Figures 2 to 6 can be retrofitted into a conventional syringe pump driver in place of the standard syringe pump shown in Figure 1.

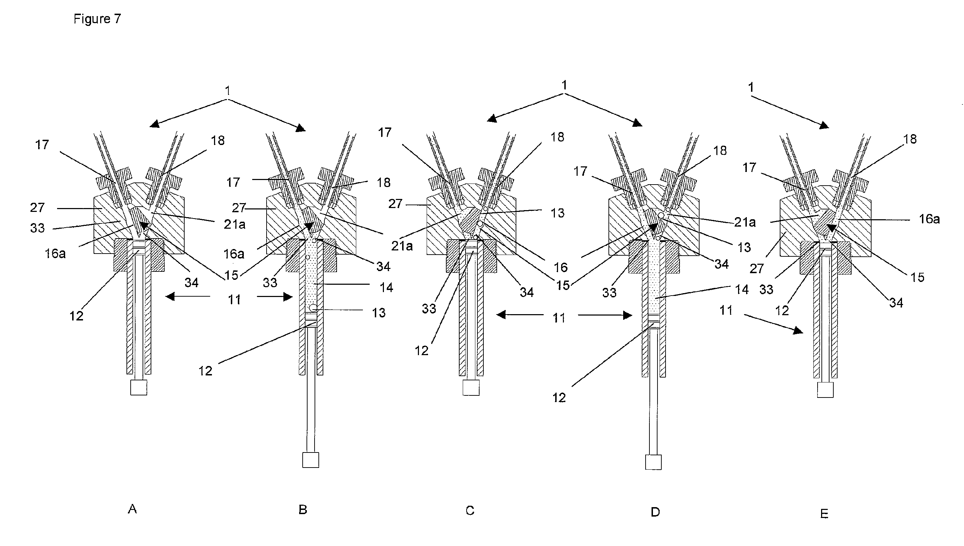

Figures 7A to 7E show the sequence of priming the syringe pump 1 comprising a rotary valve 15 as shown in Figure 4. Figure 7A shows the syringe 11 when it is not in use and the syringe 11 is empty of liquid. The cutaway section 16a is in contact with both the inlet port 17 and the syringe 11 through the port 33, whilst the cutaway portion 21a is in contact with the outlet port 18 only.

The fluid 14 enters the syringe 11 through the inlet port 17, via the cutaway section 16a of the rotary valve 15. It will be evident from Figure 7B that air bubbles 13 may be in the syringe when the liquid 14 enters.

Valve member 20 is then rotated through an intermediate state, wherein neither cutaway section is in contact with either of the ports 33, 34, and into the position shown in Figure 7C wherein the cutaway section 16a allows the syringe 11 to be used for dispensing via the outlet port 18. The air bubbles 13 move through the valve 15 ahead of the fluid and leave the syringe 11. This process may then be repeated with a water/alcohol mix in order to sterilise the liquid path.

Figure 7D shows the syringe after re-filling with liquid 14 to be dispensed. The air bubbles 13 do not re-enter the syringe 11 and therefore do not introduce inconsistencies in the volume dispensed at some later time.

Figure 7E shows the syringe pump 1 at the end of the priming sequence. All air has been purged from the syringe pump 1 and only liquid remains in the passageways 16.

All of the views of the dispenser shown in Figure 7 demonstrate the syringe 11 configured vertically. Therefore, the inlet port 17 and outlet port 18 are separated horizontally, thus providing a standard configuration of the syringe 11. The vertical configuration of the syringe ensures that bubbles rise more quickly to the top of the syringe in order to allow efficient removal of the bubbles from the syringe. Alternatively, the syringe 11 may be configured horizontally with the outlet 18 above the inlet 17. In this configuration any trapped bubbles 13 will still rise towards the port 34 and therefore pass through the valve 15 and through the outlet 18.

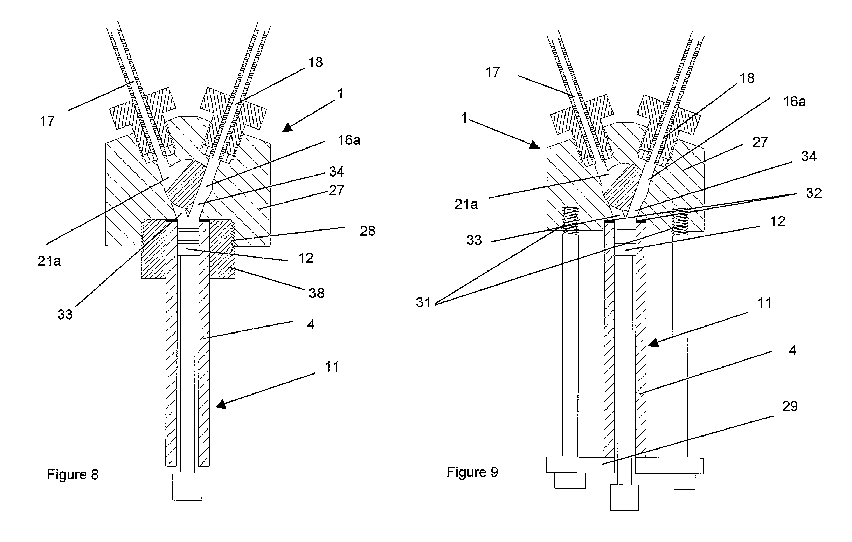

Figures 8 and 9 show two different methods of attaching a syringe 11 to a valve block 27. A particular advantage of the syringe pump 1 of the present invention is that it can be retrofitted to an existing syringe pump driver in order to improve the system performance at minimum cost. Figure 8 shows the syringe 11 attached to the valve block 27 by a support block 38 in which the top end of the barrel is fixed, for example, by adhesive, and has a conventional screw-thread 28 engaging a complementary screw-thread in the valve block 27. Figure 9 shows a clamp plate 29 at the end of the syringe barrel 4 opposite the valve. The plate 29 and associated bolts 31 which screw into the valve block 27 apply pressure to the syringe barrel 4 which, in turn, compresses a seal 32 between the syringe barrel 4 and the valve block 27. The clamp plate 29 has the advantage that it provides far better visibility of the whole length of the syringe barrel 4, in use.

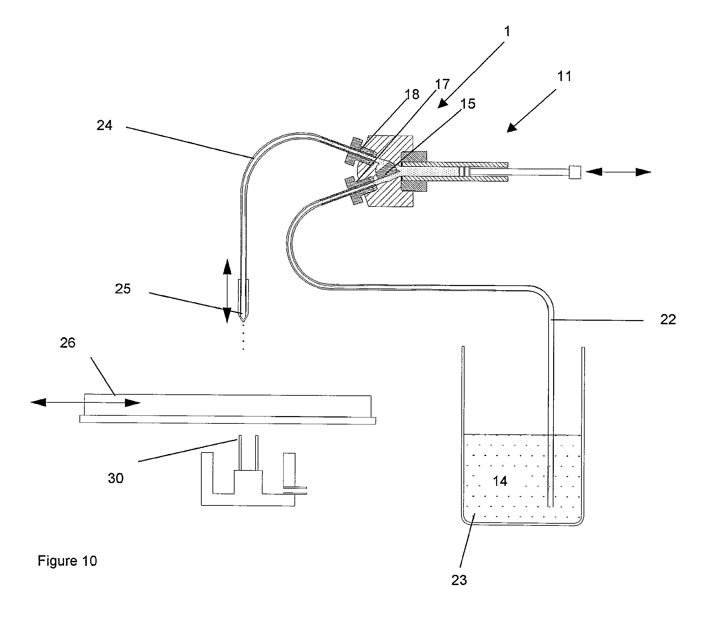

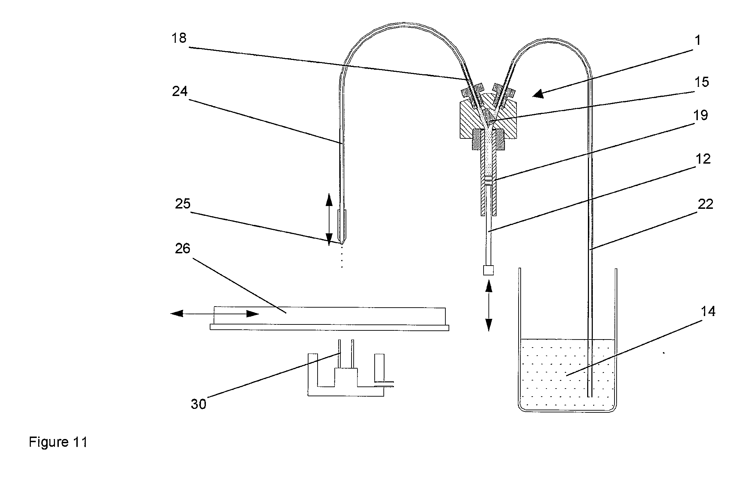

Figures 10 and 11 show further detail of the syringe pump 1 in a dispensing assembly. Figure 10 shows a horizontally configured syringe pump1 and Figure 11 shows a vertically configured syringe pump 1. The inlet 17 leads, via liquid input tubing 22, to a liquid reservoir 23 containing a fluid 14 to be dispensed. The outlet 18 leads, via liquid output tubing 24, to a dispensing tip 25 from which fluid 14 is dispensed into a conventional multi-well microplate 26.

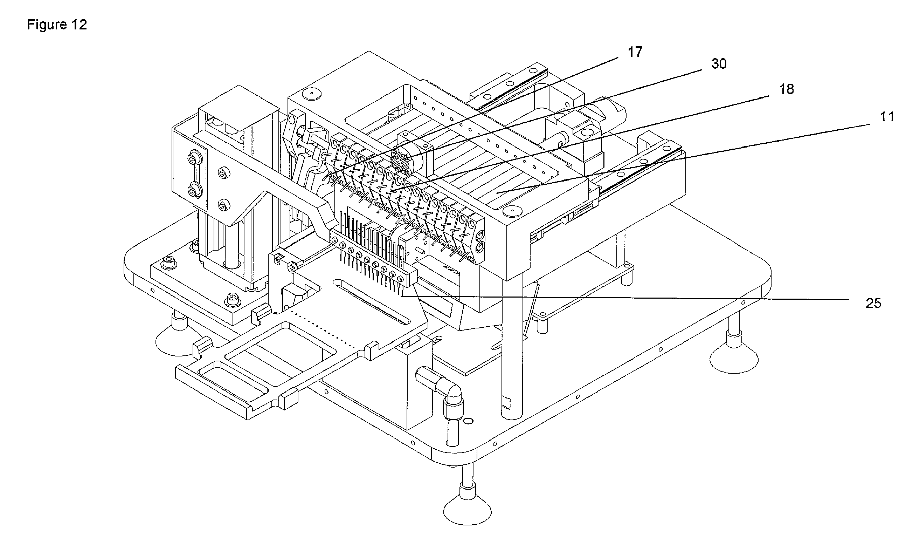

A dispensing array can be formed from a plurality of dispensing assemblies as shown in Figure 12. The dispensing tips 25 of the dispensers are formed into a substantially parallel dispensing array and the inlets 17 are connected via a manifold 30 to the reservoir (not shown). For clarity, the input and output tubing connecting each inlet/outlet 17, 18 to the manifold 30 and dispensing tip 25 has been omitted. Furthermore, the syringes 11 are also formed into a substantially parallel array such that the valve core is common across all syringe pumps 1 and syringe plungers 12 can be actuated simultaneously by an actuator moving in a plane perpendicular to the plane of the array of plungers 12.

## Claims

1. A syringe pump (1) comprising:
a barrel (4) and a movable plunger (12),
a valve (15) provided at one end of the barrel,
a first port (33) between the valve (15) and the barrel (4),
a second port (34) between the valve and the barrel (4), and
wherein the valve (15) has
a first inlet/outlet (17),
a second inlet/outlet (18); and
a valve member (20) including at least one valve passage (16) configured to connect the first and second ports (33, 34) selectively to the first and second inlet/outlets (17, 18) depending on the position of the valve member (20).

2. A syringe pump (1) according to claim 1, wherein the valve member (20) is a rotary valve member and the valve passage (16) is located across the rotational axis of the valve member (20).

3. A syringe pump (1) according to claim 1, wherein the valve member is a rotary valve member and the valve passage (16) is offset from the rotational axis of the valve member (20).

4. A syringe pump (1) according to claim 2 or claim 3, wherein the valve member (20) further comprises a second passage (21) offset from the rotational axis of the valve member (20).

5. A syringe pump (1) according to claim 4, wherein the passages (16, 21) are parallel to one another.

6. A syringe pump (1) according to claims 1 to 4, where the passages are non-parallel to one another.

7. A syringe pump (1) according to any of the preceding claims, wherein the or each passage (16, 21) is a bore through the valve member (20).

8. A syringe pump (1) according to any of claims 1 to 6, wherein the or each passage (16a, 21a) is provided by a recess as a cut-away portion in the side of the valve member (20).

9. A syringe pump (1) according to claim 1, wherein the valve member is axially movable.

10. A dispensing assembly including at least one syringe pump (1) according to any of claims 1 to 9 and a dispenser (10) having:
a dispensing tip (25) connected to the outlet (18) of the or each syringe pump (1).

11. A dispensing array comprising a plurality of dispensers (10) according to claim 10, wherein the dispensing tips (25) and syringe plungers (12) are configured to form substantially parallel arrays.

12. A dispensing array according to claim 11, further comprising an actuator configured to actuate the syringe plungers (12) simultaneously.
